# EUROPÄISCHE PATENTANMELDUNG

(11) **EP 1 338 333 A1**
(43) Veröffentlichungstag der Anmeldung: **27.08.2003**
(21) Anmeldenummer: 03002618.1
(22) Anmeldetag: 10.02.2003
(51) Int. Cl.: B01J 3/04, B01J 8/04

(54) **Reaktorkaskade aus Haupt- und Nachreaktor**

(30) Priorität: 14.02.2002 DE 10206132
(71) Anmelder: BASF AKTIENGESELLSCHAFT, 67056 Ludwigshafen (DE)
(72) Erfinder: Zehner, Peter, Dr., 67071 Ludwigshafen (DE); Herwig, Stephan, Dr., 67071 Ludwigshafen (DE)
(74) Vertreter: Isenbruck, Günter, Dr.

(57) **Zusammenfassung**

Reaktorkaskade aus Haupt- und Nachreaktor umfassend einen geschlossenen Hauptreaktorbehälter und einen mit dem Hauptreaktorbehälter verbundenen geschlossenen Nachreaktorbehälter, wobei der Hauptreaktorbehälter mindestens eine Eduktzuleitung und der Nachreaktorbehälter mindestens eine Produktableitung und gegebenenfalls eine oder mehrere weitere Eduktzuleitungen aufweist, wobei der Nachreaktorbehälter sich im Innern des Hauptreaktorbehälters oder der Hauptreaktorbehälter sich im Innern des Nachreaktorbehälters befindet und der eine Reaktorbehälter von dem anderen Reaktorbehälter umschlossen wird.

## Beschreibung

Die Erfindung betrifft eine Reaktorkaskade aus einem Haupt- und einem Nachreaktor.

Hochdruckreaktoren sind verhältnismäßig teuere Apparate. Der hohe Betriebsdruck macht große Wandstärken erforderlich und bringt zudem ein erhöhtes Gefahrenpotential mit sich. Konstruktion, Montage und Wartung sowie die wiederkehrenden Prüfungen sind daher erheblich aufwendiger als bei drucklos betriebenen Reaktoren.

Häufig ist es aus Gründen der Reaktionsführung - beispielsweise um ein bestimmtes Verweilzeitverhalten von Edukten und Produkten im Reaktionsgemisch zu realisieren - notwendig, die Reaktion zweistufig in einem Hauptreaktor und einem Nachreaktor ablaufen zu lassen. Ist die betreffende Reaktion eine Druckreaktion, so stellt sich bei derartigen zweistufigen Reaktorkaskaden das Problem, dass beide Reaktoren - Haupt- und Nachreaktor - für den betreffenden Reaktionsdruck ausgelegt sein müssen.

JP 47/16382 offenbart einen Reaktionsapparat, der einen inneren und einen konzentrischen äußeren Reaktoren umfasst. Innerer und äußerer Reaktor haben getrennte Ein- und Auslässe und sind nicht miteinander verbunden. In einem Reaktor wird eine exotherme Reaktion durchgeführt, während in dem anderen Reaktor gleichzeitig eine endotherme Reaktion durchgeführt wird. Die Anordnung der Reaktoren, bei der der innere Reaktor von dem äußeren Reaktor umschlossen wird (die innere Wand des äußeren Reaktors ist gleichzeitig die äußere Wand des inneren Reaktors), ermöglicht einen guten Wärmeübergang von der exothermen zu der endothermen Reaktion.

Aufgabe der Erfindung ist es, eine Reaktorkaskade aus Haupt- und Nachreaktor bereitzustellen, die besonders wirtschaftlich ist.

Gelöst wird die Aufgabe durch eine Reaktorkaskade aus Haupt- und Nachreaktor umfassend einen geschlossenen Hauptreaktorbehälter und einen mit dem Hauptreaktorbehälter verbundenen geschlossenen Nachreaktorbehälter, wobei der Hauptreaktorbehälter mindestens eine Eduktzuleitung aufweist und der Nachreaktorbehälter mindestens eine Produktableitung und gegebenenfalls eine oder mehrere weitere Eduktzuleitungen aufweist, dadurch gekennzeichnet, dass der Nachreaktorbehälter sich im Innern des Hauptreaktorbehälters oder der Hauptreaktorbehälter sich im Innern des Nachreaktorbehälters befindet und der eine Reaktorbehälter von dem anderen Reaktorbehälter umschlossen wird.

Die erfindungsgemäße Reaktorkaskade weist gegenüber aus dem Stand der Technik bekannten Reaktorkaskaden den Vorteil auf, dass nur ein Druckbehälter erforderlich ist. Der innere Reaktor braucht nicht als Druckapparat ausgeführt zu werden, da der Außenmantel des äußeren Reaktors den Betriebsdruck trägt. Wird der innere Reaktor bei einem anderen Druck als der äußere Reaktor betrieben, so muss der Außenmantel des inneren Reaktors nur für die Druckdifferenz zwischen innerem und äußerem Reaktor ausgelegt sein.

In einer Ausführungsform der erfindungsgemäßen Reaktorkaskade befindet sich der Nachreaktorbehälter im Innern des Hauptreaktorbehälters und wird von diesem umschlossen.

Da die erfindungsgemäße Reaktorkaskade nur einen Druckbehälter erfordert, werden erhebliche Kosten für Material, Fundamente, Anschlüsse, Rohrleitungen, Wartung und Überwachung eingespart. Ferner beansprucht die erfindungsgemäße Reaktorkaskade weniger Platz als eine herkömmliche Reaktorkaskade.

Bevorzugt sind Hauptreaktor und Nachreaktor als konzentrische Rohrreaktoren ausgebildet.

In den erfindungsgemäßen Reaktorkaskaden können Reaktionen in flüssiger Phase oder als zweiphasige Gas/Flüssig-Reaktionen durchgeführt werden, gegebenenfalls an einem homogen oder heterogen vorliegenden Katalysator.

In einigen Ausführungsformen der Erfindung sind die erfindungsgemäßen Reaktorkaskaden so ausgestaltet, dass in ihnen Gas/Flüssig-Reaktionen durchgeführt werden können, also Reaktionen, bei denen ein Reaktant in der Gasphase und ein weiterer Reaktant in flüssiger Phase vorliegt. Dabei können äußerer und innerer Reaktor unabhängig voneinander in Sumpf- oder Rieselfahrweise betrieben werden.

Bei katalytischen Reaktionen in Sumpffahrweise kann der Katalysator als in der flüssigen Phase gelöster Homogenkatalysator oder als Heterogenkatalysator im Festbett oder Wirbelbett vorliegen. Bei katalytischen Reaktionen in Rieselfahrweise liegt der Katalysator im allgemeinen im Festbett angeordnet vor.

Die Erfindung wird nachstehend mit Bezugnahme auf die Zeichnungen näher erläutert.

In einer Ausführungsform der Erfindung ist die Reaktorkaskade als Reaktorkaskade zur Durchführung von Gas/Flüssig-Reaktionen in Sumpf/Sumpf-Fahrweise ausgestaltet und weist mindestens eine Zuleitung zur Einspeisung eines flüssigen Eduktstroms am Sumpf des Hauptreaktorbehälters, mindestens eine Zuleitung mit Gasverteiler zur Einspeisung eines Gasstroms am Sumpf des Hauptreaktorbehälters, mindestens eine Leitung mit Verteiler zum Transport des Flüssigkeitsstroms und des Gasstroms vom Kopf des Hauptreaktorbehälters zum Sumpf des Nachreaktorbehälters und eine Produktableitung am Kopf des Nachreaktors auf.

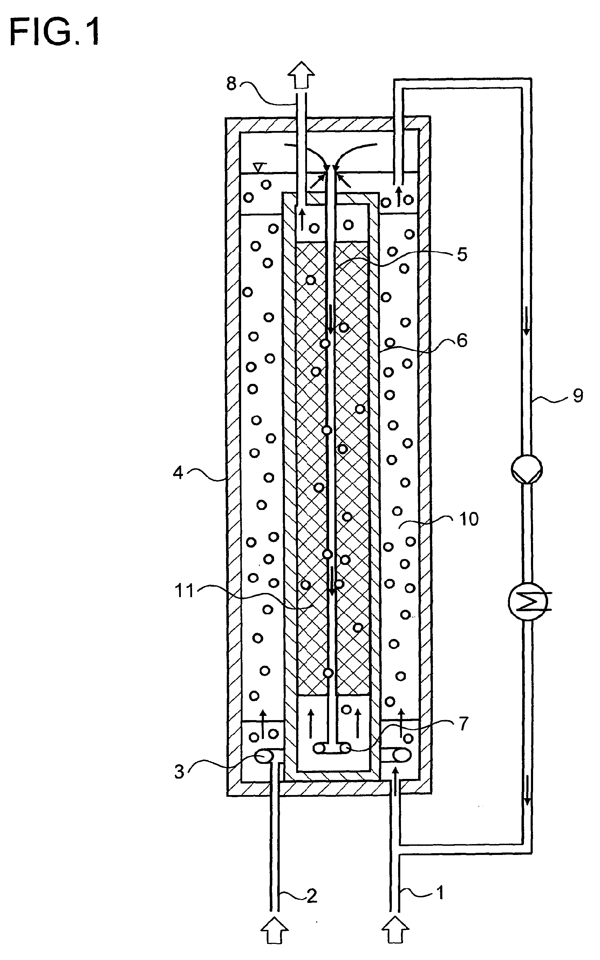

Figur 1 zeigt beispielhaft eine derartige Reaktorkaskade zur Durchführung von Gas/Flüssig-Reaktionen in Sumpf/Sumpf-Fahrweise. Ein flüssiger Eduktstrom wird über die Zuleitung 1 eingespeist, ein Gasstrom wird über die Zuleitung 2 in den Hauptreaktorbehälter 4 eingespeist. Über den Gasverteiler 3 wird der gasförmige Reaktant in der flüssigen Phase dispergiert und steigt in dieser in Form von Gasblasen auf. Der Gasverteiler 3 kann, wie in Figur 1 angedeutet, als Begasungsring ausgestaltet sein. Durch die Leitung 5, die vorzugsweise als einfaches Fallrohr ausgestaltet ist, werden Gas und Flüssigkeit zum Sumpf des Nachreaktorbehälters 6 transportiert. Der Ringverteiler 7 dient zur möglichst guten Verteilung der Gasphase in der flüssigen Phase. Am Kopf des Nachreaktorbehälters wird über die Leitung 8 ein Produktstrom entnommen. Ein Teil der flüssigen Phase kann am Kopf des Hauptreaktorbehälters entnommen werden und über die Umpumpkreislaufleitung 9 wieder dem Sumpf des Hauptreaktorbehälters zugeführt werdeb. In Haupt- und Nachreaktor sind Katalysatorfestbetten 10 bzw. 11 angeordnet.

Haupt- und Nachreaktor können auch als einfache Blasensäulen ohne Katalysatorfestbett ausgestaltet sein. In diesem Fall liegt der Katalysator vorzugsweise als Homogenkatalysator in der flüssigen Phase vor.

In einer weiteren Ausführungsform der Erfindung ist die Reaktorkaskade als Reaktorkaskade zur Durchführung von Gas/Flüssig-Reaktionen in Riesel/Sumpf-Fahrweise ausgestaltet und weist mindestens eine Zuleitung mit einem Flüssigkeitsverteiler zur Einspeisung eines flüssigen Eduktstroms am Kopf des Hauptreaktorbehälters, mindestens eine Leitung zur Einspeisung eines Gasstroms in den Hauptreaktorbehälter, mindestens eine Verbindung zwischen Sumpf des Hauptreaktorbehälters und Sumpf des Nachreaktorbehälters zum Transport des Flüssigkeitsstroms vom Sumpf des Hauptreaktorbehälters zum Sumpf des Nachreaktorbehälters, mindestens eine Zuleitung mit Gasverteiler zur Einspeisung eines Gasstroms am Sumpf des Nachreaktorbehälters und mindestens eine Produktableitung am Kopf des Nachreaktorbehälters auf.

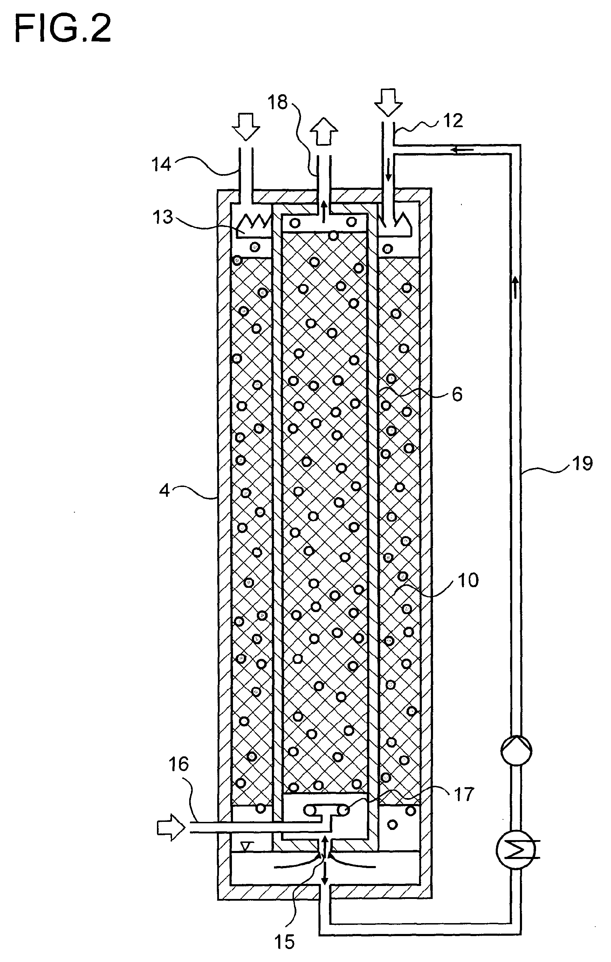

Figur 2 zeigt beispielhaft eine derartige Reaktorkaskade zur Durchführung von Gas/Flüssig-Reaktionen in Riesel/Sumpf-Fahrweise. Der flüssige Eduktstrom wird über die Zuleitung 12 und den Flüssigkeitsverteiler 13 in den Hauptreaktorbehälter 4 eingespeist und rieselt über das Katalysatorfestbett 10. Der Flüssigkeitsverteiler ist vorzugsweise als Überlauf-Verteiler ausgestaltet. Der gasförmige Reaktant wird über die Zuleitung 14 an beliebiger Stelle, vorzugsweise am Kopf in den Hauptreaktorbehälter 4 eingespeist. Durch die Öffnung 15 gelangt die flüssige Phase, angetrieben durch den im Hauptreaktor herrschenden Überdruck, in den Nachreaktorbehälter 6. Am Sumpf des Nachreaktorbehälters wird über die Zuleitung 16 und den Gasverteiler 17 zusätzlicher gasförmiger Reaktant eingespeist und in der flüssigen Phase dispergiert. Am Kopf des Nachreaktorbehälters wird über die Leitung 18 Produkt entnommen. Am Sumpf des Hauptreaktorbehälters wird ein Teil der flüssigen Phase entnommen und über die Umpumpkreislaufleitung 19 dem Flüssigkeitsverteiler am Kopf des Hauptreaktorbehälters 4 zugeführt.

In einer weiteren Ausführungsform der Erfindung ist die Reaktorkaskade als Reaktorkaskade zur Durchführung von Gas/Flüssig-Reaktionen in Riesel/Riesel-Fahrweise ausgestaltet und weist mindestens eine Zuleitung mit Flüssigkeitsverteiler zur Einspeisung eines flüssigen Eduktstroms am Kopf des Hauptreaktorbehälters, mindestens eine Zuleitung zur Einspeisung eines Gasstroms in den Hauptreaktorbehälter, mindestens eine Leitung mit Flüssigkeitsverteiler zum Transport des Flüssigkeitsstroms vom Sumpf des Hauptreaktorbehälters zum Kopf des Nachreaktorbehälters, mindestens eine Zuleitung zur Einspeisung eines Gasstroms in den Nachreaktorbehälter und mindestens eine Produktableitung am Sumpf des Nachreaktorbehälters auf.

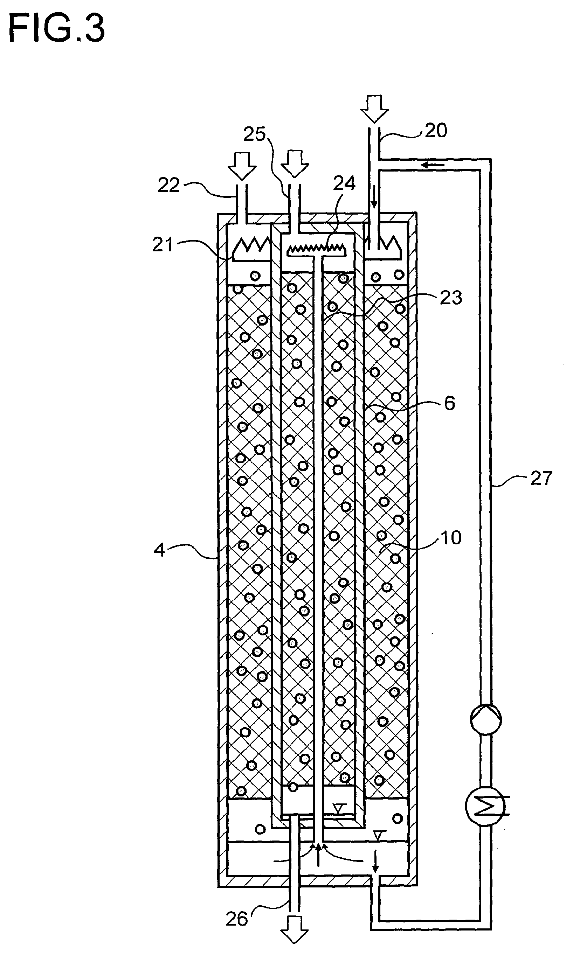

Figur 3 zeigt beispielhaft eine derartige Reaktorkaskade zur Durchführung von Gas/Flüssig-Reaktionen in Riesel/Riesel-Fahrweise. Der flüssige Eduktstrom wird über die Zuleitung 20 und den Flüssigkeitsverteiler 21 in den Hauptreaktorbehälter 4 eingespeist und rieselt über das Katalysatorfestbett 10. Der gasförmige Reaktant wird über die Zuleitung 22 an beliebiger Stelle, vorzugsweise am Kopf in den Hauptreaktorbehälter 4 eingespeist. Durch die vorzugsweise als einfaches Steigrohr ausgebildete Leitung 23 gelangt die flüssige Phase, angetrieben durch den im Hauptreaktor herrschenden Überdruck, zum Flüssigkeitsverteiler 24 am Kopf des Nachreaktorbehälters 6. Über die Zuleitung 25 wird zusätzlicher gasförmiger Reaktant an beliebiger Stelle, vorzugsweise am Kopf, in den Nachreaktorbehälter eingespeist. Am Sumpf des Nachreaktorbehälters wird über die Leitung 26 Produkt entnommen. Am Sumpf des Hauptreaktorbehälters wird ein Teil der flüssigen Phase entnommen und über die Umpumpkreislaufleitung 27 dem Flüssigkeitsverteiler am Kopf des Hauptreaktorbehälters zugeführt.

In einer weiteren Ausführungsform der Erfindung ist die Reaktorkaskade als Reaktorkaskade zur Durchführung von Gas/Flüssig-Reaktionen in Sumpf/Riesel-Fahrweise ausgestaltet und weist mindestens eine Zuleitung zur Einspeisung eines flüssigen Eduktstroms am Sumpf des Hauptreaktorbehälters, mindestens eine Zuleitung mit Gasverteiler zur Einspeisung eines Gasstroms am Sumpf des Hauptreaktorbehälters, mindestens eine Leitung mit Flüssigkeitsverteiler zum Transport des Flüssigkeitsstroms vom Kopf des Hauptreaktorbehälters zum Kopf des Nachreaktorbehälters, mindestens eine Zuleitung zur Einspeisung eines Gasstroms in den Nachreaktorbehälter und mindestens eine Produktableitung am Sumpf des Nachreaktorbehälters auf.

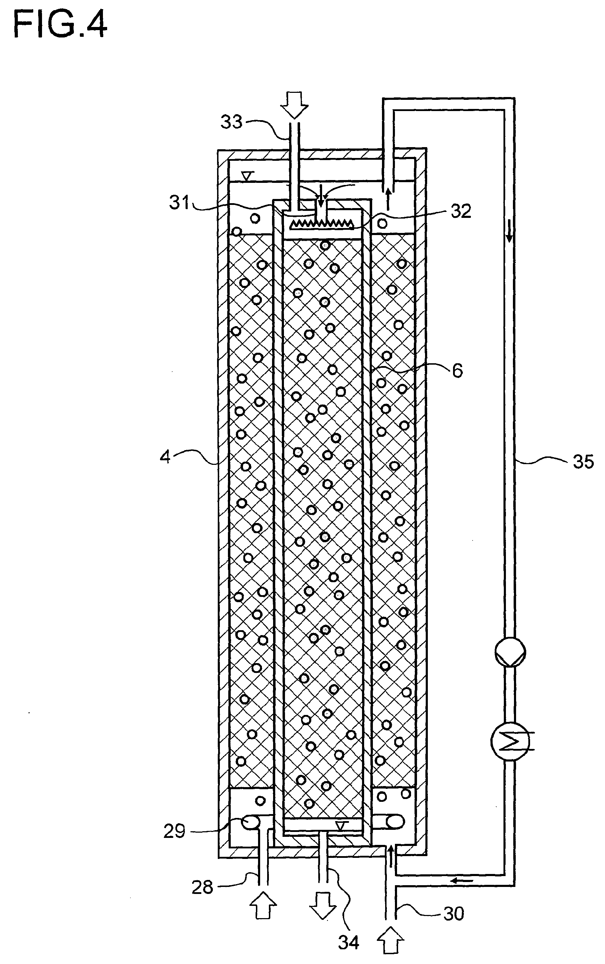

Figur 4 zeigt beispielhaft eine derartige Reaktorkaskade zur Durchführung von Gas/Flüssig-Reaktionen in Sumpf/Riesel-Fahrweise. Der Gasstrom wird über die Zuleitung 28 und den Gasverteiler 29 in den Hauptreaktorbehälter 4 eingespeist und in der flüssigen Phase dispergiert. Durch die Zuleitung 30 wird der Flüssigkeitsstrom eingespeist. Durch die Leitung 31 wird die flüssige Phase zum Flüssigkeitsverteiler 32 am Kopf des Nachreaktorbehälters 6 transportiert und rieselt von dort über das Katalysatorfestbett 10. Über die Leitung 33 wird zusätzlicher gasförmiger Reaktant an beliebiger Stelle, vorzugsweise am Kopf in den Nachreaktor eingespeist. Am Sumpf des Nachreaktors wird über die Leitung 34 ein Produktstrom entnommen. Ein Teil der flüssigen Phase wird am Kopf des Hauptreaktorbehälters entnommen und über die Umpumpkreislaufleitung 35 wieder dem Sumpf des Hauptreaktorbehälters zugeführt.

Die erfindungsgemäße Reaktorkaskade aus Haupt- und Nachreaktor ist aus den im Reaktorbau üblichen Materialien wie Edelstahl, z. B. mit der Werkstoffnummer 1.4541 oder Cr/Mo-Stahl gefertigt.

Gas/Flüssig-Reaktionen, die in den erfindungsgemäßen Reaktorkaskaden aus Haupt- und Nachreaktor durchgeführt werden können, sind üblicherweise Hochdruck-Hydrierungen, die mit den üblichen dafür geeigneten Homogen- oder Heterogenkatalysatoren und unter den üblichen Hydrierbedingungen durchgeführt werden. Beispiele für Hydrierungen sind die Herstellung von Sorbitol, von Hexandiol sowie allgemein Hydrierungen von Oxo-Aldehyden zu Oxo-Alkoholen.

In Frage kommen aber auch andere üblicherweise unter hohem Druck durchgeführte Synthesen wie Hydroformylierungen, beispielsweise die Hydroformylierung von Polyisobuten zu Oxo-Polyisobuten in Gegenwart von Cobaltcarbonylwasserstoff.

## Patentansprüche

1. Reaktorkaskade aus Haupt- und Nachreaktor umfassend einen geschlossenen Hauptreaktorbehälter und einen mit dem Hauptreaktorbehälter verbundenen geschlossenen Nachreaktorbehälter, wobei der Hauptreaktorbehälter mindestens eine Eduktzuleitung und der Nachreaktorbehälter mindestens eine Produktableitung und gegebenenfalls eine oder mehrere weitere Eduktzuleitungen aufweist, **dadurch gekennzeichnet, dass** der Nachreaktorbehälter sich im Innern des Hauptreaktorbehälters oder der Hauptreaktorbehälter sich im Innern des Nachreaktorbehälters befindet und der eine Reaktorbehälter von dem anderen Reaktorbehälter umschlossen wird.

2. Reaktorkaskade nach Anspruch 1, **dadurch gekennzeichnet, dass** Hauptreaktor und Nachreaktor als konzentrische Rohrreaktoren ausgebildet sind.

3. Reaktorkaskade nach Anspruch 1 oder 2 zur Durchführung von Gas/Flüssig-Reaktionen in Sumpf/Sumpf-Fahrweise, **gekennzeichnet durch** mindestens eine Zuleitung zur Einspeisung eines flüssigen Eduktstroms am Sumpf des Hauptreaktorbehälters, mindestens eine Zuleitung mit Gasverteiler zur Einspeisung eines Gasstroms am Sumpf des Hauptreaktorbehälters, mindestens eine Leitung mit Verteiler zum Transport des Flüssigkeitsstroms und des Gasstroms vom Kopf des Hauptreaktorbehälters zum Sumpf des Nachreaktorbehälters und eine Produktableitung am Kopf des Nachreaktorbehälters.

4. Reaktorkaskade nach Anspruch 1 oder 2 zur Durchführung von Gas/Flüssig-Reaktionen in Riesel/Sumpf-Fahrweise, **gekennzeichnet durch** mindestens eine Zuleitung mit einem Flüssigkeitsverteiler zur Einspeisung eines flüssigen Eduktstroms am Kopf des Hauptreaktorbehälters, mindestens eine Leitung zur Einspeisung eines Gasstroms in den Hauptreaktorbehälter, mindestens eine Verbindung zwischen Sumpf des Hauptreaktorbehälters und Sumpf des Nachreaktorbehälters zum Transport des Flüssigkeitsstroms vom Sumpf des Hauptreaktorbehälters zum Sumpf des Nachreaktorbehälters, mindestens eine Zuleitung mit Gasverteiler zur Einspeisung eines Gasstroms am Sumpf des Nachreaktorbehälters und mindestens eine Produktableitung am Kopf des Nachreaktorbehälters.

5. Reaktorkaskade nach Anspruch 1 oder 2 zur Durchführung von Gas/Flüssig-Reaktionen in Riesel/Riesel-Fahrweise, **gekennzeichnet durch** mindestens eine Zuleitung mit Flüssigkeitsverteiler zur Einspeisung eines flüssigen Eduktstroms am Kopf des Hauptreaktorbehälters, mindestens eine Zuleitung zur Einspeisung eines Gasstroms in den Hauptreaktorbehälter, mindestens eine Leitung mit Flüssigkeitsverteiler zum Transport des Flüssigkeitsstroms vom Sumpf des Hauptreaktorbehälters zum Kopf des Nachreaktorbehälters, mindestens eine Zuleitung zur Einspeisung eines Gasstroms in den Nachreaktorbehälter und mindestens eine Produktableitung am Sumpf des Nachreaktorbehälters.

6. Reaktorkaskade nach Anspruch 1 oder 2 zur Durchführung von Gas/Flüssig-Reaktionen in Sumpf/Riesel-Fahrweise, **gekennzeichnet durch** mindestens eine Zuleitung zur Einspeisung eines flüssigen Eduktstroms am Sumpf des Hauptreaktorbehälters, mindestens eine Zuleitung mit Gasverteiler zur Einspeisung eines Gasstroms am Sumpf des Hauptreaktorbehälters, mindestens eine Leitung mit Flüssigkeitsverteiler zum Transport des Flüssigkeitsstroms vom Kopf des Hauptreaktorbehälters zum Kopf des Nachreaktorbehälters, mindestens eine Zuleitung zur Einspeisung eines Gasstroms in den Nachreaktorbehälter und mindestens eine Produktableitung am Sumpf des Nachreaktorbehälters.

7. Reaktorkaskade nach einem der Ansprüche 1 bis 6, **dadurch gekennzeichnet, dass** Haupt- und Nachreaktorbehälter ein Katalysatorfestbett enthalten.

8. Verwendung der Reaktorkaskade nach einem der Ansprüche 1 bis 7 zur Durchführung von Hydrierungen oder Hydroformylierungen.

9. Verwendung nach Anspruch 8 zur Herstellung von Sorbitol, zur Herstellung von Hexandiol, zur Hydrierung von Oxo-Aldehyden und zur Hydroformylierung von Polyisobuten.
